(19) Europäisches Patentamt
European Patent Office
Office européen des brevets

(11) EP 0 877 605 B1

# (12) EUROPÄISCHE PATENTSCHRIFT

(45) Veröffentlichungstag und Bekanntmachung des Hinweises auf die Patenterteilung:
**16.10.2002 Patentblatt 2002/42**

(21) Anmeldenummer: **97902218.3**

(22) Anmeldetag: **24.01.1997**

(51) Int Cl.[7]: **A61K 9/20**

(86) Internationale Anmeldenummer:
**PCT/EP97/00329**

(87) Internationale Veröffentlichungsnummer:
**WO 97/026867 (31.07.1997 Gazette 1997/33)**

## (54) FESTE INSTANT-RELEASE-DARREICHUNGSFORMEN UND VERFAHREN ZU IHRER HERSTELLUNG

SOLID INSTANT-RELEASE FORMS OF ADMINISTRATION AND PROCESS FOR PRODUCING THE SAME

FORMES SOLIDES D'ADMINISTRATION A LIBERATION INSTANTANEE ET LEUR PROCEDE DE PREPARATION

(84) Benannte Vertragsstaaten:
**AT BE CH DE DK ES FI FR GB GR IE IT LI LU NL PT SE**
Benannte Erstreckungsstaaten:
**RO SI**

(30) Priorität: **26.01.1996 DE 19602757**

(43) Veröffentlichungstag der Anmeldung:
**18.11.1998 Patentblatt 1998/47**

(73) Patentinhaber: **Heidelberg Pharma Holding GmbH**
**69126 Heidelberg (DE)**

(72) Erfinder:
- **GABEL, Rolf-Dieter**
  **D-68723 Schwetzingen (DE)**
- **WIRL, Alexander**
  **D-67259 Heuchelheim (DE)**
- **WOOG, Heinrich**
  **D-69514 Laudenbach (DE)**

(74) Vertreter: **Patentanwälte Zellentin & Partner**
**Rubensstrasse 30**
**67061 Ludwigshafen (DE)**

(56) Entgegenhaltungen:

| | |
|---|---|
| **WO-A-92/03462** | **WO-A-92/10172** |
| **US-A- 4 164 560** | **US-A- 4 283 394** |
| **US-A- 4 605 551** | **US-A- 4 622 392** |
| **US-A- 4 999 189** | **US-A- 5 223 263** |

- **CHEMICAL ABSTRACTS, vol. 110, no. 14, 3.April 1989 Columbus, Ohio, US; abstract no. 121307, HIRAI S. ET AL: "Formulation studies of a new oral cephalosporin, cefotiam hexetil hydrochloride" XP002036381 & YAKUZAIGAKU, Bd. 48, Nr. 3, 1988, Seiten 189-196, HIRAI S. ET AL: "Formulation studies of a new oral cephalosporin, cefotiam hexetil hydrochloride"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 877 605 B1

## Beschreibung

Gegenstand der vorliegenden Erfindung sind feste Instant-Release-Darreichungsformen (IR-Darreichungsformen) umfassend therapeutische Wirkstoffe oder Wirkstoffkonzentrate, insbesondere Lipidkonjugate von Nukleosiden, die in wäßrigen Medien die Eigenschaften besitzen, Gele auszubilden., Die Erfindung betrifft ferner Verfahren zur Herstellung derartiger Instant-Release-Formen.

Arzneisubstanzen (Wirkstoffe) werden nur in den seltensten Fällen ohne Formgebung verwendet. Die Überführung in feste IR-Darreichungsformen mit üblichen galenischen Verfahren wird bekanntermaßen durch Verwendung von Hilfsstoffen ermöglicht.

Der Zerfall von IR-Darreichungsformen soll in der Regel sehr rasch erfolgen, damit daraus die geforderten hohen Auflösungsraten des Wirkstoffes in vitro erzielt werden können. Der rasche Zerfall der Darreichungsform wird auf der einen Seite durch die Auswahl der Hilfsstoffe und des Herstellverfahrens bestimmt, auf der anderen Seite aber auch durch das Auflöseverhalten des Wirkstoffes selbst. Die eingesetzten Wirkstoffe sollten beim Auflösen in wäßrigen Medien üblicherweise keine Gele oder gelähnliche Strukturen ausbilden, damit ein gegenseitiges Verkleben der einzelnen Wirkstoffleilchen so weit wie möglich ausgeschlossen werden kann.

Es gibt aber auch zahlreiche Wirkstoffe mit lipophilem Rest, z.B. Wirkstoffe aus der Gruppe der Lipid-Konjugate, die beim Verarbeiten zu festen IR-Darreichungsformen mit den herkömmlichen galenischen Verfahren beim Auflösen in wäßrigen Medien zur Ausbildung von Gelen oder gelähnliche Strukturen neigen und dadurch keine ausreichend rasche Auflösungsraten des Wirkstoffes in vitro erreicht werden. Dieses gilt auch für Wirkstoffverschnitte, die den Wirkstoff in hoher Konzentration enthalten (Wirkstoffkonzentrate). Dieser Nachteil wird besonders dann deutlich, wenn monolithische Darreichungsformen (z.B. Tabletten) in hohen Dosierungen gewünscht werden. Insbesondere bei hohen Dosierungen nimmt hierbei der beschriebene Effekt mit steigender Wirkstoffkonzentration zu. Die In-vitro-Auflösungsrate dieser Formulierungen und mithin auch die Auflösungsgeschwindigkeit und Resorptionsgeschwindigkeit in vivo sind gegenüber flüssigen Darreichungsformen stark vermindert.

Unter dem Begriff gel-bildende Wirkstoffe werden im Sinne der Erfindung solche Wirkstoffe verstanden, die in Konzentrationen von weniger als 20 % (w/v-%) in wässrigen Systemen bei 20 °C gelartige Phasen ausbilden, und die hierbei resultierenden Lösungen kein Newtonsches Fließverhalten aufweisen. Als Newtonsche Flüssigkeiten werden solche Flüssigkeiten bezeichnet, deren durch die Newtonsche Gleichung $t=h \cdot D$ definierter Fließwiderstand bei gegebener Temperatur eine Stoffkonstante ist, wobei t die Schubspannung ist, D das Geschwindigkeitsgefälle und h die dynamische Viskosität bedeutet.

Solche Wirkstoffe und deren Herstellung sind beispielsweise in den Anmeldungen WO 92/03462; WO 93/16092; WO 93/16091; WO 94/03465; PCT/EP94/02123; DE 4402492; DE 4418690; sowie beispielsweise in WO 91/19726; EP 0 350 287; US 5,223,263; US 5,194,654; US 4,921,951; US 4,622,392; US 4,291,024; US 4,283,394 beschrieben. Im Falle von antiviral wirksamen Nukleosid-Derivaten werden in EP 0 350 287 und US 5,223,263 Lipid-Derivate (Diacylglycerol-Nukleoside) und deren Verwendung in liposomaler Form beschrieben.

Auch mit Hilfe von einem erheblichen Zusatz an Hilfsstoffen konnten die häufig auch hygroskopischen, instabilen, mit vielen gängigen Hilfsstoffen inkompatiblen und in wäßrigen Medien zu starker Gelbildung neigenden Wirkstoffe nicht zu festen, rasch zerfallenden Darreichungsformen verarbeitet werden. Beim Einbringen der Wirkstoff-Hilfsstoff-Mischungen bzw. der daraus hergestellten Darreichungsformen in ein wäßriges Freisetzungsmedium bildet sich in der Grenzphase sofort eine hochviskose Gelschicht aus, die eine weitere rasche Auflösung unmöglich macht. Diese Gelschichten lösen sich nur sehr langsam ab, ähnlich wie bei der Hydrokolloidmatrix, und bewirken somit einen unerwünschten retardierenden Effekt.

Dieser retardierende Effekt kann zwar durch Verdünnung mit geeigneten Hilfsstoffen teilweise ausgeglichen werden, führt aber wegen der benötigten hohen Mengen an Hilfsstoffen zu keiner der Dosierung entsprechenden Tablettengröße, bzw. bei höheren Wirkstoffdosierungen sind monolithische Darreichungsformen nicht mehr herstellbar.

Desweiteren hängt die In-vitro-Auflösungsrate bei gleicher Rezeptur vom Komprimierungsgrad in der Darreichungsform ab, so daß eine ausreichende In-vitro-Auflösungsrate bei komprimierten Darreichungsformen (z.B. Tabletten) bzw. auch im Falle von Kapselfüllmassen nicht erreichbar war. Außerdem weisen entsprechende hergestellte Tabletten häufig unzureichende Härten und zu hohe Abriebe auf, so daß bei der Herstellung von mit Filmen überzogenen Tabletten hohe Verluste in Folge des Zerbrechens der Tabletten in den verwendeten Dragierkesseln eintreten.

Ein weiterer Nachteil ergibt sich durch das konventionelle Herstellungsverfahren. Durch die Verteilung des Wirkstoffes in den zur wäßrigen Granulation benötigten Hilfsstoffen wird der Wirkstoff auch in Kontakt mit inkompatiblen Hilfsstoffen gebracht und somit kann häufig keine ausreichend lange Haltbarkeit der Darreichungsform erzielt werden. Außerdem wurde festgestellt, daß die homogene Verteilung des hygroskopischen Wirkstoffes in der Darreichungsform nicht immer gewährleistet wird, weil die Wirkstoffpartikel im feuchten Milieu schnell zu größeren Einheiten agglomerieren. Eine inhomogene Verteilung des Wirkstoffes in der pharmazeutischen Mischung mit anderen Hilfsstoffen ist jedoch problematisch, da bei der Weiterverarbeitung zu Einzeldosisformen, wie beispeilsweise Tabletten oder Kapseln, unterschiedliche Wirkstoffmengen in der Darreichungsform resultieren können. Zusätzlich resultierten im Rahmen der

technischen Produktion von größeren Mengen trotz gleicher Rezepturzusammensetzung und gleicher Verfahrensschritte von Charge zu Charge unterschiedliche In-vitro-Auflösungsraten der einzelnen Darreichungsformen, die über den zulässigen bzw. üblichen Schwankungsbreiten lagen. Im Hinblick auf die gebotene Arzneimittelsicherheit sind jedoch bei der Entwicklung von Arzneimitteln derartige Risiken weitgehend auszuschließen.

Aus den genannten Gründen war es nicht möglich, auf dem üblichen Wege mit den galenischen Standardmethoden Abmischen, Granulation, Sprühtrocknung, Sprüherstarrung oder Preßgranulierung des Wirkstoffes mit genügend Hilfsstoffen zu geeigneten Einzel-Darreichungsformen, beispielsweise Tabletten, in der gewünschten Dosierung zu kommen, und die Darreichungsformen über eine hinreichend rasche In-vitro-Auflösungsrate verfügen. Zudem beeinflußt der Granulataufbau die Wirkstoffstabilität negativ, d.h. mit konventionellen Methoden konnte keine befriedigende Lösung erreicht werden.

Das US-P 4,605,551 beschreibt öl-umhüllte Antacida-Partikel. Antacida besitzen teilweise ungünstige sensorische Eigenschaften und können auch stark agglomeriert sein. Zur Verbesserung dieser negativen Eigenschaften wird der Wirkstoff mit einem wasserunlöslichen Öl, das einen elektronegativen Zusatzstoff enthält, gleichmäßig überzogen, um die elektropositive Ladung des Wirkstoffs aufzuheben (Desagglomerierung). Diese Mischung kann dann problemlos zu einer Darreichungsform weiterverarbeitet werden. So wird in Beispiel 3 Öl-umhülltes Aluminiumhydroxid-Gel ( 50 % Anteil) mit einer 10 %igen wässrigen Stärkepaste und weiteren Zusatzstoffen granuliert. Diese Stoffe weisen jedoch bei weniger als 20 % (g/g) Feststoffanteil in Wasser keine gelbildenden Eigenschaften (< 5 mPas+s) auf, darüber hinaus ist der Umhüllungsstoff flüssig und entfaltet keine die Gelbildung hemmenden oder kompensierenden Eigenschaften.

Gemäß WO92/10172 A1 wird ein spezieller Wirkstoff (Almokalant) vor Weiterverarbeitung in eine Immediate-Release- oder Extended-Release-Darreichungsform an einen Polystyrolsulfonatkomplex gebunden. Dadurch werden verschiedene Eigenschaften wie z.B. Geruch oder Bindungseigenschaften verbessert sowie die Stabilität erhöht. Almokalant ist eine viskose, klebrige und instabile pharmakologisch aktive Substanz und kein in Wasser quellender bzw. gelbildender Wirkstoff.

Das US-P 4,164,568 beschreibt Systeme zur Freisetzung von hydrophilen Makromolekülen aus unlöslichen polymeren Matrizes. Es handelt sich um ein neuartiges Retard-System zur gleichmäßigen Freisetzung über einen langen Zeitraum. Das Quellen der Makromoleküle (biologischen Materialien) in Wasser ist dafür die Voraussetzung, wodurch in der Polymermatrix Kanäle und Mikroporen erzeugt werden, die die Freisetzungsgeschwindigkeit bestimmen. Instant Release Formulierungen lassen sich so nicht herstellen.

Im US-P 5,223,263 werden Wirkstoffe beschrieben, die zur Verbesserung ihrer Resorptionseigenschaften in Lipsomen eingebaut sind und zu gängigen Darreichungsformen weiterverarbeitet werden. Liposomen sind wässrige, ggf. den Wirkstoff enthaltende Kompartimente, die von einer ein- oder mehrschichtigen Lipidmembran umgeben sind. Die Größe der Partikel beträgt in der Regel um die 50 nm und setzt eine Wirkstoffparkikelgröße im Nanobereich voraus, so daß Wirkstoffpartikel mit Durchmessern > 10 µm so nicht verarbeitet werden können.

Aus US-P 4,622,392 sind Thiophospholipidkonjugate zytotoxischer Wirkstoffe und deren Verarbeitung zu üblichen galenischen Formulierungen bekannt. Es wird weder die Zugabe von Hilfsstoffen beschrieben, die die Gelbildung stark quellender Wirkstoffe herabsetzen sollen, noch gibt es Hinweise auf in Wasser stark quellende Wirkstoffe selbst.

Von Hirai et al., CA vol. 48, Nr. 3, 1988, S. 189 - 196 ist die Verhinderung einer Gelbildung des antibakteriellen Wirkstoffs Cephalosporin durch Zusatz von Zitronensäure und Cyclodextrinen beschrieben. Eine gelhemmende Wirkung wird mit der Zitronensäure erreicht. Die Cyclodextrine schließen die Wirkstoffe in wechselnden Mengen in den innermolekularen Kanälen ein (molekulare Verkapselung).

Der Erfindung lag deshalb die Aufgabe zugrunde, verbesserte IR-Darreichungsformen von Wirkstoffen oder Wirkstoffkonzentraten zu entwickeln, die in wäßrigen Medien Gele ausbilden.

Die Aufgabe der Erfindung wird durch eine IR-Darreichungsform gelöst, bei der gelbildende Wirkstoffe oder Wirkstoffkonzentrate in einer quellregulierenden Umhüllung aus kompatiblen Hilfsstoffen eingebettet sind, die die Gelbildung hemmen oder kompensieren.

Überraschend wurde gefunden, daß eine ausgewählte Gruppe von Hilfsstoffen geeignet ist, die Gelbildung von Wirkstoffen oder Wirkstoffkonzentraten in wäßrigen Medien im Sinne der Erfindung zu reduzieren bzw. zu hemmen oder zu kompensieren. Darüber hinaus sind derartige Darreichungsformen stabil und ausreichend lange haltbar, ohne daß Zersetzungsprodukte des Wirkstoffes oder der verwendeten Hilfsstoffe auf Basis möglicher Wechselwirkungen zwischen Wirkstoff und Hilfsstoffen nachweisbar sind. Geeignete Hilfsstoffe im Sinne der Erfindung sind Makromoleküle, wie z.B. Polyvinylpyrrolidone, Gelatine, Gelatinederivate, Stärken, Stärkederivate, Cellulosen, Cellulosederivate, Macrogole, Polyvinylalkohole und Polyacrylsäuren, sowie Hilfsstoffe aus der Gruppe der Zucker, Zuckeralkohole, Glyceride, Salze von Fettsäuren, Fette, Wachse, Tenside, Silikate oder hochdisperses Siliciumdioxid, sowie deren Kombinationen. Insbesondere kommen die Hilfsstoffe hochdisperses Siliciumdioxid, Polyvinylpyrrolidon, Cellulosen und Zucker in Frage. Diese Hilfsstoffe sind mit den Wirkstoffen kompatibel. Sie können einzeln oder in Kombination die quellregulierende Umhüllung bilden. Die Umhüllung der Wirkstoffpartikel ist hierbei derart, daß jedes einzelne Wirkstoffpartikel mit einer Primärhülle an Hilfsstoffen umgeben ist, wobei sich die Wirkstoffpartikel selbst nicht direkt berühren.

Bekanntermaßen können Makromoleküle (z.B. Polyvinylpyrrolidone, Gelatinen, Gelatinederivate, Stärken, Stärkederivate, Cellulosen, Cellulosederivate, Macrogole, Polyvinylalkohole und Polyacrylsäuren), Zucker, Zuckeralkohole, Salze von Fettsäuren, Fette, Wachse Tenside, Silikate, aber auch hochdisperses Siliciumdioxid mit Wasser Gele bilden bzw. führen zu einer Erhöhung der Viskosität im wäßrigen Milieu. Glyceride zeigen vergleichbare Effekte. Desweiteren ist bekannt, daß die oben genannten Wirkstoffe mit wäßrigen Medien Gele ausbilden. Aufgrund dieser Tatsachen war eigentlich zu erwarten, daß nach abgeschlossener Auflösung des Wirkstoffes in wäßrigen Medien ein Zusatz an oben genannten gel-bildenden Hilfsstoffe einen Viskositätsanstieg der Lösungen bewirkt.

Der Zusatz der genannten Hilfsstoffe in Form einer Primärhülle führt jedoch wider Erwarten zu Viskositätserniedrigungen, d.h. der an sich zu erwartende additive Effekt von Wirkstoff und Hilfsstoff, der zu eimem Anstieg der Viskosität führen sollte, tritt nicht ein. Überraschenderweise wurde gefunden, daß die gewünschten raschen In-vitro-Auflösurigsraten erreicht werden. Außerdem weisen die komprimierten Darreichungsformen (Tabletten) die notwendigen, erwünschten physikalischen Eigenschaften wie ausreichende Härte und geringen Abrieb auf.

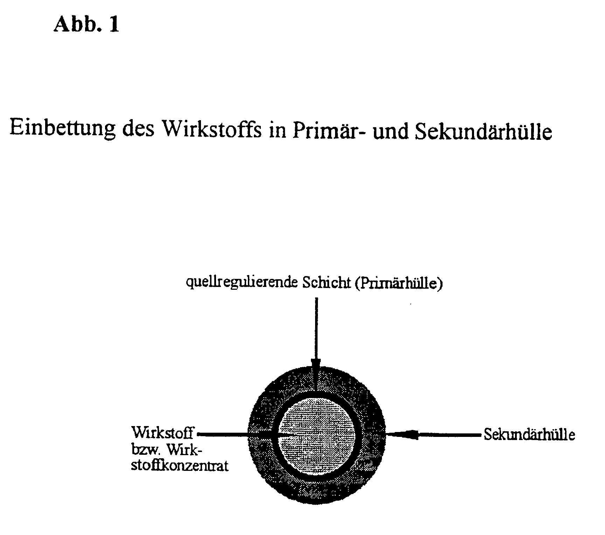

In einer bevorzugten Ausführungsform der Erfindung können die Wirkstoffe oder Wirkstoffkonzentrate zusätzlich zur quellregulierenden Primärhülle auch von einer weiteren Umhüllung (Sekundärhülle) umgeben sein (s. Abb. 1). Zur sekundären Umhüllung oder Einbettung der mit einer Primärumhüllung versehenen Partikel sind die gleichen Hilfsstoffe wie für die Primärumhüllung einzeln oder in Kombination geeignet.

Die erfindungsgemäße feste IR-Darreichungsform kann entweder aus Partikeln mit Primärumhüllung (Primärpartikel), Partikeln mit Primärumhüllung und Sekundärumhüllung (Sekundärpartikel) oder aus Primärpartikeln (innere Phase) mit einer äußeren Phase bzw. aus Sekundärpartikeln (innere Phase) mit einer äußeren Phase bestehen. Gegenstand der äußeren Phase sind solche Hilfsstoffe, die zur Herstellung von Tabletten ausgehend von den Wirkstoff/Hilfsstoff-Granulaten geeignet sind. Die äußere Phase beinhaltet pharmazeutisch gebräuchliche Hilfsstoffe, wie Füllmittel und/oder Zerfallhilfsmittel und/oder Fließ-, Schmier- oder Trennmittel, wie z.B. mikrokristalline Cellulose, Natriumcarboxymethylstärke, quervernetztes Polyvinylpyrrolidon, Siliciumdioxid oder Tenside.

Erfindungsgemäß liegt das Verhältnis von Wirkstoff zu Hilfsstoff für die IR-Darreichungsform bei einer Primärumhüllung zwischen 1:0,01 und 1:100, bevorzugt zwischen 1:0,05 und 1:5. In IR-Darreichungsformen mit Primär- und Sekundärhülle liegt das Gewichtsverhältnis von Wirkstoff zu Hilfsstoff für die Primärhülle im Bereich von 1:0,01 bis 1:10 und für die Sekundärhülle im Bereich von 1:0,1 bis 1:100, bevorzugt 1:1 bis 1:10. Der Wirkstoffgehalt der erfindungsgemäßen IR-Darreichungsform beträgt 0,5 - 90 %, vorzugsweise 5 - 50 %. Erfindungsgemäß liegt die durchschnittliche Korngröße (d') des Wirkstoffs oder Wirkstoffkonzentrates im Bereich von 10 µm - 3 mm. Bevorzugte Untergrenzen sind 50 µm, 100 µm oder 200 µm. Die Obergrenzen liegen bevorzugt bei 500 µm, 700 µm oder 1 mm.

Als gel-bildende Wirkstoffe im Sinne der vorliegenden Erfindung werden solche Wirkstoffe verstanden, die beim Auflösen in Wasser oder in pufferhaltigen wässrigen Systemen bei Konzentrationen von weniger als 20 %, vorzugsweise bei etwa 2 - 10 %, eine Viskositätserhöhung der Lösungen bewirken. Die Viskosität derartiger Lösungen liegt bei Werten oberhalb von 5 mPas*sec, insbesondere bei mehr als 100 mPas*sec, vorzugsweise mehr als 500 mPas*sec.

In wäßrigen Medien gelbildende Wirkstoffe bzw. deren Salze oder Wirkstoffkonzentrate gemäß der Erfindung sind beispielsweise Verbindungen der allgemeinen Formel I:

$$L - B - D \qquad (I),$$

wobei D eine pharmakologisch aktive Substanz (drug), L einen lipophilen Rest und B eine die Gruppen L und D verbindende Linkergruppe darstellt.

Insbesondere bedeutet B eine Brücke $-O-[(PO)(OH)O]_n-$ mit n = 0, 1, 2, 3 und L ein Lipidteil der allgemeinen Formel II

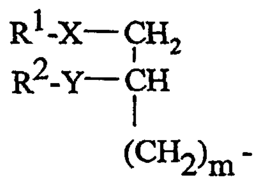

(II)

in dem

$R^1$ eine geradkettige oder verzweigte, gesättigte oder ungesättigte Alkylkette mit 1 - 30 Kohlenstoffatomen ist, die

gegebenenfalls ein- oder mehrfach durch $C_3$-$C_8$-Cycloalkyl- oder gegebenenfalls substituierte Phenylgruppen, Halogen, $C_1$-$C_6$-Alkoxy, $C_1$-$C_6$- Alkylmercapto-, $C_1$-$C_6$-Alkoxycarbonyl-, $C_1$-$C_6$-Alkylsulfinyl- oder $C_1$-$C_6$-Alkylsulfonylgruppen substituiert sein kann,

$R^2$ Wasserstoff, eine geradkettige oder verzweigte, gesättigte oder ungesättigte Alkylkette mit 1 - 20 Kohlenstoffatomen ist, die gegebenenfalls ein- oder mehrfach durch Halogen, $C_1$-$C_6$-Alkoxy, $C_1$-$C_6$-Alkylmercapto-, $C_1$-$C_6$-Alkoxycarbonyl-, oder $C_1$-$C_6$-Alkylsulfonylgruppen substituiert sein kann,

X einen Valenzstrich, Sauerstoff, Schwefel, Oxycarbonyl, Carbonyloxy, Carbonylamido, Amidocarbonyl, die Sulfinyl- oder die Sulfonylgruppe darstellt,

Y einen Valenzstrich, Oxycarbonyl, Carbonyloxy, Carbonylamido, Amidocarbonyl, ein Sauerstoff- oder Schwefelatom ist, und

m eine ganze Zahl zwischen 1 und 5 darstellt.

In der allgemeinen Formel II bedeutet $R^1$ vorzugsweise eine geradkettige oder verzweigte $C_8$-$C_{15}$-Alkylgruppe, die noch durch eine $C_1$-$C_6$-Alkoxy- oder eine $C_1$-$C_6$-Alkylmercaptogruppe substituiert sein kann. $R^1$ ist insbesondere eine gesättigte Alkylkette, die gegebenenfalls durch eine $C_3$-$C_8$-Cycloalkyl- oder eine gegebenenfalls substituierte Phenylgruppe substituiert ist. $R^1$ stellt insbesondere eine Nonyl-, Decyl-, Undecyl-, Dodecyl-, Tridecyl-, Tetradecyl-, Cyclohexyl-hexyl- oder Phenyl-hexylgruppe dar, wobei die Phenylgruppe gegebenenfalls durch $C_1$-$C_6$-Alkyl oder Halogen substituiert ist. Als $C_1$-$C_6$-Alkoxy-substituenten von $R^1$ kommen vorzugsweise die Methoxy-, Ethoxy-, Butoxy und die Hexyloxygruppen in Frage. Ist $R^1$ durch einen $C_1$-$C_6$-Alkylmercaptorest substituiert, versteht man darunter insbesondere den Methylmercapto-. Ethylmercapto-, Propylmercapto-, Butylmercapto- und den Hexylmercaptorest.

$R^2$ bedeutet vorzugsweise eine geradkettige oder verzweigte $C_8$-$C_{15}$-Alkylgruppe, die noch durch eine $C_1$-$C_6$-Alkoxygruppe oder eine $C_1$-$C_6$-Alkylmercaptogruppe substituiert sein kann. $R^2$ stellt insbesondere eine Octyl-, Nonyl-, Decyl-, Undecyl-, Dodecyl-, Tridecyl- oder Tetradecylgruppe dar. Als $C_1$-$C_6$-Alkoxysubstituenten von $R^2$ kommen vorzugsweise die Methoxy-, Ethoxy-, Propoxy-, Butoxy- und die Hexyloxygruppe in Frage. $R^2$ bedeutet insbesondere eine $C_8$-$C_{15}$-Alkylgruppe, vorzugsweise eine Octyl-, Nonyl-, Decyl-, Undecyl-, Dodecyl-, Tridecyl- oder Tetradecylgruppe.

Ist $R^2$ durch einen $C_1$-$C_6$-Alkylmercaptorest substituiert, versteht man darunter insbesondere den Methylmercapto-, Ethylmercapto-, Butylmercapto- und Hexylmercaptorest.

X ist bevorzugt gleich Schwefel, Sulfinyl oder Sulfonyl und Y gleich Sauerstoff. Die Heteroatome X und Y im Lipidteil L können in Ausnahmefällen durch die aus Lecithin bekannten Carbonsäureester ersetzt werden, da sonst häufig schon im Serum oder in der Leber (first pass-effect) eine hydrolytische Spaltung zu den entsprechenden Lysolecithin-Derivaten oder Glycerolestern mit entsprechend schnellerer Elimination der pharmakologisch aktiven Substanz erfolgen würde. Die Thioether- bzw. Etherlipide (X, Y = 0,S) zeigen diese Spaltung im Serum verschiedener Spezies, inklusive des Menschen, nicht.

Bevorzugt sind auch Verbindungen, in denen X und Y einen Valenzstrich darstellen, $R^2$ gleich Wasserstoff ist und $R^1$ eine $C_1$-$C_{30}$-Alkylkette darstellt, die gegebenenfalls durch $C_1$-$C_6$-Alkoxy, oder $C_1$-$C_6$-Alkylmercapto substituiert sein kann.

m ist bevorzugt gleich 1 oder 2 und besonders bevorzugt gleich 1.

Für den Fall, daß die zuvor genannten Phenylgruppen substituiert sind, kommen als Substituenten vorzugsweise Halogen, $C_1$-$C_6$-Alkyl, $C_1$-$C_6$-Alkoxy, $C_1$-$C_6$-Alkylmercapto-, $C_1$-$C_6$-Alkoxycarbonyl- oder $C_1$-$C_6$-Alkylsulfonylgruppen in Frage.

Die Brücke B wird durch die Formel

$$-0-[(PO)(OH)O]_n-$$

umschrieben, in der n = 0, 1, 2 oder 3 sein kann, aber bevorzugt gleich 0, 1 oder 2 ist und insbesondere 1 ist.

Der Lipidteil L und die Phosphatbrücke B haben die oben angegebene Bedeutung, wobei L bevorzugt einen Rest der Formel II darstellt und B bevorzugt eine Phosphatbrücke ist. Besonders bevorzugt ist eine Phosphatbrücke mit n = 1 und ein Lipidteil der Formel II, in dem $R^1$ und $R^2$ einen Alkylrest mit 8 - 15 C-Atomen darstellen, X gleich Schwefel und Y gleich Sauerstoff ist.

Der Begriff "pharmakologisch aktive Substanz" (Bezeichnung D in Formel I) steht für einen Wirkstoff im arzneimittelrechtlichen Sinne. Dieser Wirkstoff kann ein Wirkstoff eines bereits eingeführten, von Arzneimittelbehörden zugelassenen Pharmazeutikums oder ein sich in der arzneimittelrechtlichen Zulassung oder Entwicklung befindlicher Wirkstoff sein. Die Definition "pharmakologisch aktive Substanz" umfaßt auch solche Derivate von Wirkstoffen, die durch Einführung einer oder mehrerer fünktioneller Gruppen (beispielsweise solche Gruppen, die eine Kupplung von D mit dem Lipid-Carrier-Teil L ermöglichen, wie z.B. Hydroxy- oder Aminogruppen) chemisch modifiziert werden können. Die Definition umfaßt ferner die aus dem Wirkstoff D sich - bildenden Prodrugformen, die ebenfalls physiologisch aktiv sind. Insbesondere kommen solche pharmakologisch aktive Substanzen D in Frage, deren klinische Entwicklung

aufgrund unerwünschter Nebenwirkungen eingestellt oder nicht begonnen wurde, bzw. die über ein sehr enges Dosis-Wirkungs-Spektrum verfügen, so daß die Verabreichung der therapeutisch erforderlichen Menge nur mit hohen Risiken oder praktisch überhaupt nicht beherrschbar war.

Es ist bekannt, daß die therapeutische Breite einer pharmakologisch aktiven Substanz signifikant verbessert wird, wenn die Substanz an ein lipidartiges Trägermolekül gekoppelt wird. Das so hergestellte Konjugat dient als neuer Wirkstoff für die Herstellung von pharmazeutischen Darreichungsformen. Insgesamt resultiert aus der Kopplung eine verstärkte Wirkung der pharmazeutisch aktiven Substanz D in vivo, da durch das entstehende Drug-Delivery-Transport-System eine Lokalisierung der pharmakologisch aktiven Substanz in Zielzellen erfolgt und dadurch die pharmakologisch aktive Substanz hinsichtlich ihrer Effizienz gesteigert wird. Dies bedeutet, daß einerseits die Menge der zu verabreichenden pharmakologisch aktiven Substanz reduziert werden kann, oder andererseits unter Beibehaltung der gleichen effektiven Menge eine verstärkte pharmakologische Wirkung erzielt wird.

Die den pharmakologisch aktiven Substanzen D zugrunde liegende chemische Struktur kann ferner derart modifiziert werden, daß die Substanzen hinsichtlich ihrer physikalischen oder chemischen Eigenschaften verändert sind und beispielsweise eine höhere oder geringere Lipophilie aufweisen, jedoch hinsichtlich ihrer therapeutischen Wirkung im wesentlichen die gleichen Eigenschaften besitzen wie die unmodifizierte Substanz D. Insbesondere ist es vorteilhaft, wenn die Substanz D durch Einführung von funktionellen Gruppen chemisch derart modifiziert wird, daß sie über eine geeignete Brücke an den Lipidteil L gekoppelt werden kann. Dies geschieht beispielsweise durch Einführung von Hydroxygruppen, die über die Phosphatgruppe B an das Lipid gekoppelt werden. Für den Fall, daß der Wirkstoff bereits eine Phosphatgruppe besitzt, wie beispielsweise im Fall des Wirkstoffes Foscarnet (HO-P(O)(OH) -COOH), kann diese auch direkt zur Kopplung an das Lipid verwendet werden. In derartigen Fällen bedeutet n in der Definition von B vorzugsweise die Zahl 0.

Die pharmakologisch aktive Substanz D stellt eine chemische oder auf biologischer Basis (Antikörper, Peptid, Protein, Hormon, Toxin etc.; INDEX NOMINUM, International Drug Directory, Medpharm) aufgebaute Substanz mit biologischer Wirkung dar, sowie deren durch Einführung einer fünktionellen Gruppe (z.B. einer Hydroxygruppe) chemisch modifizierten Derivate.

Im Sinne der Erfindung kommen insbesondere alle in vitro wirksamen, jedoch in vivo im therapeutischen Bereich toxischen pharmakologisch aktive Substanzen in Frage, d.h. alle Substanzen mit kleiner therapeutischer Breite, die über eine chemisch funktionelle Gruppe zur Knüpfung der kovalenten Bindung zum Phosphat verfügen. Außerdem können auch solche Substanzen verwendet werden, die in ihrer pharmakologisch aktiven Form zunächst keine funktionelle Gruppe enthalten, diese sich jedoch durch chemische Modifikation einführen läßt, ohne daß ein Verlust der Wirkung der Substanz eintritt.

Bevorzugt werden solche pharmakologisch aktiven Substanzen zur Konjugation mit einem Lipidrest L verwendet, die normalerweise nach Phosphorylierung ihre aktive Form erreichen (wie z.B. im Fall von Nukleosiden). Aus dem Konjugat wird dann das pharmakologisch aktive Substanzphosphat durch enzymatische Hydrolyse des Konjugats freigesetzt. Die Freisetzung der phosphorylierten Substanz ist insbesondere deshalb von Bedeutung, da dieser Prozeß auch in solchen Zellen stattfinden kann, die nicht über die normalerweise zur Aufphosphorylierung der reinen pharmakologisch aktiven Substanz nötigen Enzyme (Kinasen) verfügen. Die konjugierte und durch Spaltung mittels intrazellulär vorhandener Enzyme freigesetzte pharmakologisch aktive Substanz kann beispielsweise eine zytostatische, zytotoxische, antitumorale, antivirale, antiretrovirale, immunsupprimierende oder immunstimulierende Wirkung aufweisen.

Für den Fall, daß die Verbindungen der Formel I protonenabspaltende Reste enthalten, wie z.B. eine oder mehrere Carboxy-, Phosphat- oder Sulfonylgruppen, können im Sinne der Erfindung auch die entsprechenden Ester mit Alkoholen, sowie die pharmakologisch verträglichen Salze dieser Säuren, wie z.B. deren Alkali- oder Erdalkalisalze, eingesetzt werden. Die entsprechenden Ester sind insbesondere $C_1$-$C_6$-Alkylester, wie z.B. die Methyl- oder Ethylester. Pharmakologisch verträgliche Salze sind insbesondere die Natrium- und Kaliumsalze.

Als pharmakologisch aktive Substanzen D kommen solche Verbindungen in Frage, die gegebenenfalls durch Einführung einer funktionellen Gruppe, die die Wirkung nicht signifikant beeinflußt, in ein kopplungsfähiges Derivat überführt werden, das dann z.B. das Tumorwachstum verlangsamt, eine in die DNA und/oder RNA interkalierende Substanz ist, die Topoisomerase I und II hemmt, ein Tubulinhemmer ist, ein Alkylanz ist, eine die Ribosomen inaktivierende Verbindung ist, ein Tyrosinphosphokinase-Inhibitor ist, ein Differenzierungsinduktor ist, ein Hormon, Hormonagonist oder Hormonantagonist ist, eine Substanz ist, welche die pleiotrope Resistenz gegenüber Zytostatika verändert, ein Calmodulin-Inhibitor ist, ein Proteinkinase C-Inhibitor ist, ein P-Glycoprotein-Inhibitor ist, ein Modulator der mitochondrial gebundenen Hexokinase ist, ein Inhibitor der $\gamma$-Glutamylcysteinsynthetase oder der Glutathion-S-Transferase ist, ein Inhibitor der Superoxiddismutase ist, ein Inhibitor der Reversen Transkriptase von HIV- 1 und -2 ist.

Die pharmakologisch aktive Substanz D kann eine antiinflammatorische, antirheumatische, antiphlo-gistische, analgetische oder antipyretische Wirkung aufweisen. Sie kann ferner ein Antiarrhythmikum, Calciumantagonist, Antihistaminikum, ein Hemmer der Phosphodiesterase oder ein Sympathomimetikum bzw. Parasympathomimetikum sein.

Als pharmakologisch aktive Substanzen D kommen alle Substanzen in Frage mit kurzer Halbwertszeit, insbesondere auch Verbindungen mit unterschiedlicher Organ-, Gewebe-, oder Zellhalbwertszeiten, mit schlechter Bioverfügbarkeit, d.h mit schlechter Resorption, hoher Leberspaltung oder schneller Elimination-, mit schlechter Membranpenetration (z.B, Zellmembran, Blut-Hirnschranke) mit Knochenmarktoxizität oder anderen limitierenden Organtoxizitäten (z.B. Cardio-, Leber-, Nephro-, Neurotoxizität etc.), deren Wirkkonzentration in vivo zu gering ist. Außerdem sind solche Substanzen geeignet, die gezielt mit dem Zellkern der Zielzellen in Wechselwirkung treten und auf der Ebene der DNA oder RNA in das molekulare Geschehen eingreifen, wie z.B. Antisense-Oligonukleotide, DNA-Fragmente, und die für die Gentherapie verwendet werden können.

Pharmakologisch aktive Substanzen D in der Formel I sind beispielsweise: AZT (Azidothymidin), FLT (Fluorthymidin), 5-FU (5-Fluoruridin), 6-MPR, Fludarabin, Cladribin, Pentostatin, ara-C, ara-A, ara-G, ara-R Acyclovir, Ganciclovir, Foscarnet, Doxorubicin, 4'-epi-Doxorubicin, 4'-Desoxy-doxorubicin, Etoposid, Daunomycin, Idarubicin, Epirubicin, Mitoxantron, Vincristin, Vinblastin, Taxol, Colchicin, Melphalan, 3'-Desoxy-2-fluoradenosin, FdA, 5-Ethinyluracil-9-β-D-arabinofuranosid, 5-Propinyluracil-9-β-Darabino-furanosid, d4T, ddU, ddI, ddA, d2T, 2'-Desoxy-2', 2'difluorcytidin, 5-Trifluormethyl-2'-desoxyuridin, 5-Chlor-2',3'-didesoxy-3'-fluoruridin, 3'-Desoxy-3'-fluor-myoinositol, Neplanocin A, Ribavirin, Myolinositol, Fialuridin, 3TC, Lamivudin, Doxifluridin, Tegafur, Hypericin, Pseudohypericin, Usevir, Famciclovir-, Penciclovir, Carvedilol, Actinomycin A, Bleomycin, Daunorubicin, Floxuridin, Mithramycin, Mitomycin C, Mitoxanthron, Streptozotocin, Vindesin, Netilmycin, Amikacin, Gentamycin, Streptomycin, Kanamvcin A, Tobramycin, Neomycin B, Plicamycin, Papamycin, Amphotericin B, Vancomycin, Foscarnet, Idoxuridin, Trifluridin, Vidarabin sowie Morphine, Prostaglandine, Leukotriene oder Cyclosporine. Femer kommen in Frage: Terfenadin, Dexamethason, Terbutalin; Prednisolon, Fenoterol, Orciprenalin, Salbutamol, Isoprenalin, Muscarin, Bupranolol Oxyphenbutazon, Östrogen, Salicylsäure, Propranotol, Ascorbinsäure, Spongiadiol, Diclofenac, Isospongiadiol, Flufenaminsäure, Digoxin, 4-Methyl-aminophenazon, Allopurinol, Theophyllin, Epoprostenol, Nifedipin, Chinin; Reserpin, Methotrexat, Chlorambucil, Spergualin, Ibuprofen, Indomethacin, Sulfasalazin, Penicillanamin, Chloroquin.

Bevorzugte pharmakologisch aktive Substanzen sind auch beispielsweise Peptide, Proteine und Oligonucleotide, wie z.B. Corticotropin, Calcitonin, Desmopressin, Gonadotropin, Goserelin, Insulin, Zypressin, beta-Melanotropin, alpha-Melantropin, Muramyldipeptid, Oxytocin, Vasopressin, FK-506, Octreotid oder Enalkiren.

Im Rahmen der Erfindung bevorzugte Wirkstoffe sind Lipidkonjugate von Nukleosiden. Besonders bevorzugt sind dabei Azidothymidin-Konjugate, Fluorthymidin-Konjugate und 5-Fluoruridin-Konjugate. Insbesondere bevorzugt sind das Natriumsalz des 3'-Azido-3'-desoxy-5'-thymidylsäure-mono[3-(dodecylthio)-2-decyloxy-propyl]-esters, das Natriumsalz des 3'-Fluor-3'-desoxy-5'-thymidylsäure-mono[3-(dodecylthio)-2-decyloxypropyl]-esters und das Natriumsalz des 5-Fluor-5'-uridylsäure-mono[3-(dodecylthio)-2-decyloxypropyl]-esters. Ein bevorzugtes Lipidkonjugat ist ferner (3-Dodecylmercapto-2-decyloxy)-propoxy-phosphinylhydroxy-ameisensäure, sowie dessen Natriumsalze und Alkylester.

Die oben erwähnten pharmakologisch aktiven Substanzen und die daraus herzustellenden Konjugate stellen nur Beispiele dar und schränken den erfindungsgemäßen Gedanken nicht ein.

Der Gehalt der gel-bildenden Wirkstoffe pro Einzeldarreichungsform, z.B. in einer Tablette, beträgt 1 - 500 mg, vorzugsweise 10 - 300 mg, insbesondere etwa 100 - 250 mg, wobei das Gewicht der Einzeldarreichungsform 1.000 mg nicht übersteigen soll. Sind die Einzeldarreichungsformen Tabletten, können diese mit filmbildenden Überzügen versehen werden, um so beispielsweise eine geschmacksregulierende Wirkung oder eine die Freisetzung des Wirkstoffes beeinflußende Wirkung zu erzielen.

Das Verfahren zur Herstellung der erfindungsgemäßen IR-Darreichungsformen erfolgt nach den folgenden Methoden:

1) Es wird die quellregulierende Primärhülle durch Abmischen, Granulation (alle Varianten der Aufbau- bzw. Abbaugranulation), bevorzugt Feucht- oder Sprühgranulation, Trockengranulation, Sprühtrocknung, Sprüherstarrung oder Preßgranulierung des Wirkstoffes oder Wirkstoffkonzentrates mit den o.g. kompatiblen Hilfsstoffen einzeln oder mit einer Kombination dieser Hilfsstoffe aufgebracht.

Die durchschnittliche Korngröße des Wirkstoffs oder Wirkstoffkonzentrats liegt dabei im bereich von 10 µm - 3 mm, vorzugsweise von 20 µm - 500 µm.

Das Verhältnis zwischen Wirkstoff und Hilfsstoff liegt dabei zwischen 1:0,01 und 1:100, bevorzugt zwischen 1:0,05 und 1:5.

2) Das Aufbringen einer Sekundärhülle erfolgt durch Abmischen, Granulation (alle Varianten der Aufbau- bzw. Abbaugranulation), bevorzugt Feucht- oder Sprühgranulation, Trockengranulation, Sprühtrocknung, Sprüherstarrung oder Preßgranulierung der unter 1) hergestellten Primärpartikel mit den genannten Hilfsstoffen einzeln oder mit einer Kombination davon.

Das Verhältnis zwischen Wirkstoff und Hilfsstoff liegt dabei zwischen 1:0,1 und 1:100, bevorzugt zwischen 1: 1 und 1:10.

Gegebenenfalls wird den Primärpartikeln oder Sekundärpartikeln eine äußere Phase, bestehend aus pharmazeutisch gebräuchlichen Hilfsstoffen, wie Füllmittel und/oder Zerfallhilfsmittel und/oder Fließ-, Schmier- oder Trennmittel in der üblichen Weise zugemischt.

Die erfindungsgemäße IR-Darreichungsform besitzt eine vorteilhafte Wirkstofffreisetzung von mehr als 35 % nach 30 Minuten bzw. mehr als 70 % nach einer Stunde. Bevorzugt liegt die Freisetzung des Wirkstoffes bei mindestens 80 - 95 % nach einer Stunde, insbesondere mindestens 90 %.

Die erfindungsgemäße IR-Darreichungsform mit Primärumhüllung der Wirkstoffpartikel hat folgende Vorteile:

1. Verhinderung oder Reduzierung des Gelaufbaues der Wirkstoffpartikel beim Auflösen in einem wäßrigen Medium, dadurch Verbesserung der In-vitro-Auflösungsrate in der endgültigen Darreichungsform.

2. Vermeidung von Verklebungen der Wirkstoffpartikeln während der Verarbeitung in einem feuchten Milieu (Granulatherstellung) und bei der späteren Auflösung der Darreichungsform im wäßrigen Milieu.

3. Schutzfunktion gegenüber wirkstoffzersetzenden Hilfsstoffen in einer eventuellen Sekundärhülle.

4. Abschirmung der Feuchte vom Wirkstoff während des Herstellungsprozeßes und bei Lagerung der Darreichungsform.

5. Erhöhung der resultierenden Härte beim Komprimat durch bessere Verzahnung des Wirkstoffes mit anderen Hilfsstoffen.

Die sekundäre Umhüllung (oder Einbettung der mit einer Primärhülle versehenen Wirkstoffpartikel) bringt weiterhin folgende zusätzliche Vorteile für die IR-Darreichungsform mit sich:

1. Signifikante Verbesserung des Zerfalls der Komprimate in die umhüllten Primärpartikel (innere Phase). Dadurch wird zuerst die Oberfläche vergrößert, bevor die Gelbildung des mit einer Primärhülle versehenen Wirkstoffpartikels einsetzt.
2. Weiterer physikalischer Schutz vor wirkstoffzersetzenden Hilfsstoffen in einer äußeren Phase.
3. Gewährleistung der Wirksamkeit der äußeren Phase (z.B. Sprengmittelwirkung).
4. Möglichkeit der Verzahnung der einzelnen umhüllten Partikeln beim Tablettieren
5. Maskierung der plastischen Wirkstoffeigenschaften, um eine einwandfreie, preßkraftunabhängige Komprimierung zu gewährleisten, mit dem Resultat einer hohen Härte und geringem Abrieb bei den entstehenden Komprimaten.

Das bevorzugte erfindungsgemäße Verfahren zur Herstellung der IR-Darreichungsformen besteht darin, daß man in einem ersten Schritt eine die Gelbildung des Wirkstoffes hemmende primäre Umhüllung auf die Wirkstoffoberfläche unter Verwendung der erfindungsgemäßen Hilfsstoffe aufbringt. Dies erfolgt vorzugsweise durch Abmischen, Granulation, Sprühtrocknung, Sprüherstarrung oder Preßgranulierung des Wirkstoffes mit den makromolekularen Hilfsstoffen und gegebenenfalls weiteren pharmazeutischen Hilfsstoffen. Die verwendeten Wirkstoffpartikel besitzen vorzugsweise einen Durchmesser von 10 - 500 µm. Die in diesem Schritt entstehenden Partikel weisen dann einen Durchmesser von mehr als 10 µm auf, insbesondere 50 - 700 µm. In einem zweiten Schritt erfolgt dann die sekundäre Umhüllung bzw. Einbettung der in dem ersten Verfahrensschritt erhaltenen Partikel unter Verwendung der erfindungsgemäßen Hilfsstoffe bzw. weiteren pharmazeutischen Zusatzstoffen. Dieser Verfahrensschritt kann ebenfalls durch Abmischen, Granulation, Sprühtrocknung, Sprüherstarrung oder Preßgranulation der Partikel ggf mit weiteren Granulierhilfsmitteln erfolgen. Die so hergestellten Partikel weisen einen zweischichtigen Aufbau auf, wobei der Partikelkern durch den Wirkstoff selbst gebildet wird, die innere Hülle (Primärhülle) aus einer Schicht oder Umhüllung von Hilfsstoffen besteht, die die erfindungsgemäßen Hilfsstoffe enthalten. In einer zweiten Hülle (Sekundärhülle), die auf der Primärhülle aufgebracht ist, sind dann die weiteren Hilfsstoffe enthalten. Das Verhältnis von Wirkstoff zu Hilfsstoff liegt im Fall der Primärhülle vorzugsweise im Bereich von 1:0,01 bis 1:10. Das Verhältnis von Wirkstoff zu Hilfsstoff in der Sekundärhülle beträgt vorzugsweise 1:0,1 bis 1:100. In einem dritten Verfahrensschritt kann dann die Zumischung der äußeren Phase (z.B. Tablettierhilfsmittel) zu den Sekundärpartikeln erfolgen, so daß die so hergestellte Tablettiermasse direkt zu Tabletten verpreßt werden kann. Die so hergestellten Tabletten können gegebenenfalls mit einem neutralen oder einem geschmacksregulierenden oder die Freisetzung des Wirkstoffes regulierenden Film überzogen werden.

Anschließend soll die Erfindung an Ausführungsbeispielen näher erläutert werden, ohne sie darauf einzuschränken.

**Beispiel 1**

Die Varianten A und B zeigen konventionell hergestellte Darreichungsformen, Variante C eine erfindungsgemäße Darreichungsform:

| Pos. | Varianten: | A | B | C |
|---|---|---|---|---|
| | | | | |
| 1) | Wirkstoff | 206 mg | 206 mg | 206 mg |
| 2) | Siliciumdioxid, hochdispers | - | - | 14 mg |
| 3) | Mikrokristalline Cellulose | 142 mg | - | - |
| 4) | Lactose | 300 mg | 442 mg | 300 mg |
| 5) | Polyvidon K 25 | 4 mg | 4 mg | 20 mg |
| 6) | Mikrokristalline Cellulose | - | - | 176 mg |
| 7) | Natriumcarboxymethylstärke | 120 mg | 120 mg | - |
| 8) | Poly(vinylpyrrolidon),querv. | - | - | 40 mg |
| 9) | Siliciumdioxid, hochdispers | 8 mg | 8 mg | 4 mg |
| 10) | Magnesiumstearat | 20 mg | 20 mg | 20 mg |
| | Endgewicht Kern | 800 mg | 800 mg | 780 mg |
| | Härte | 40 N | 28 N | 142 N |
| | Abrieb | deckeln | deckein | 0,1 % |
| Wirkstoff: Na-Salz des 3'-Azido-3'-desoxy-5'-thymidylsäure-mono[3-(dodecylthio)-2-decyloxypropyl]-esters, | | | | |

Gelbildende Eigenschaft des Wirkstoffes: Bei der Herstellung einer wässrigen 7 %-igen Lösung des Wirkstoffes beträgt die Viskosität der Lösung 500 - 600 mPa*sec (Ausgangswert: 1 mPa*sec).

In einem ersten Schritt wird die Primärhülle hergestellt, indem man die Wirkstoffpartikel 1) mit einer wässrigen Suspension des Hilfsstoffes 2) überzieht. In einem zweiten Schritt erfolgt die Herstellung der Sekundärhülle durch Feuchtgranulation der aus dem ersten Schritt erhaltenen Partikel zusammen mit den Positionen 3) - 5). Die äußere Phase wird durch Zumischen der Positionen 6) - 10) hergestellt. Anschließend werden die so erhaltenen pharmazeutischen Massen tablettiert und die Härte und der Abrieb der erhaltenen Tabletten bestimmt. Bei den Varianten A und B wird auf die Herstellung der Primärhülle verzichtet.

Das Beispiel zeigt, daß die erfindungsgemäße IR-Darreichungsform mit Primär- und Sekundärumhüllung des Wirkstoffs nicht deckelt und einen äußerst geringen Abrieb aufweist, darüber hinaus weist sie eine entschieden höhere Härte auf. In den Varianten A und B wird der gelbildende Wirkstoff mit großen Mengen an zerfallsfördernden Hilfsstoffen (mikrokristalline Cellulose und Lactose) und dem Bindemittel PVP (Polyvidon K25) konventionell zu einem Granulat verarbeitet. In Variante 3 ist der Anteil an zerfallsfördernden Hilfsstoffen um ca. ein Drittel reduziert, aber der erfindungsgemäße Hilfsstoff Aerosil (hochdisperses Siliciumdioxid) vorhanden. Ferner wird durch das verwendete Überzugsverfahren sichergestellt, daß die Wirkstoffpartikel mit einer geschlossenen Hülle (Primärhülle) umgeben sind. Nur in Variante C werden Tabletten mit guten physikalischen Eigenschaften erhalten, deren Freisetzung die gewünschte Anforderungen erfüllt. Beim Tablettieren konnte das Deckeln der Tabletten auf einen geringen Wert unterhalb von 1 % reduziert werden. Daneben zeigt die erfindungsgemäße IR-Darreichungsform bereits nach einer Stunde eine 90% ige Freisetzung des Wirkstoffs.

**Beispiel 2**

Analog zu Beispiel 1 werden folgende drei Varianten an Darreichungsformen hergestellt: Die Varianten A und B zeigen konventionell hergestellte Darreichungsformen, Variante C eine erfindungsgemäße Darreichungsform:

| Pos. | Varianten: | A | B | C |
|---|---|---|---|---|
| | | | | |
| 1) | Wirkstoff | 100 mg | 100 mg | 100 mg |
| 2) | Siliciumdioxid, hochdispers | - | - | 13 mg |
| 3) | Mikrokristalline Cellulose | 116 mg | - | - |
| 4) | Lactose | 200 mg | 316 mg | 200 mg |
| 5) | Polyvidon K 25 | 4 mg | 4 mg | 20 mg |
| 6) | Mikrokristalline Cellulose | - | - | 120 mg |
| 7) | Natriumcarboxymethylstärke | 60 mg | 60 mg | - |
| 8) | Poly(vinylpyrrolidon),querv. | - | - | 30 mg |
| 9) | Siliciumdioxid, hochdispers | 5 mg | 5 mg | 2 mg |
| 10) | Magnesiumstearat | 15 mg | 15 mg | 15 mg |
| | Endgewicht Kern | 500 mg | 500 mg | 500 mg |
| | Härte | 45 N | 17 N | 150 N |
| | Abrieb | deckeln | deckeln | < 1 % |
| Wirkstoff = R,S-(3-Dodecylmercapto-2-decyloxy)-propoxy-phosphinylameisensäure (Di-Natriumsalz bzw. Methylester) | | | | |

Das Beispiel zeigt, daß die erfindungsgemäße IR-Darreichungsform mit Primär- und Sekundärumhüllung des Wirkstoffs nicht deckelt und einen äußerst geringen Abrieb aufweist, darüber hinaus weist sie eine entschieden höhere Härte auf. Daneben zeigt die erfindungsgemäße IR-Darreichungsform bereits nach einer Stunde eine 80%-ige Freisetzung des Wirkstoffs.

**Beispiel 3**

Analog zu Beispiel 1 werden folgende Darreichungsformen hergestellt, wobei die Varianten A und B konventionell hergestellte Darreichungsformen und Variante C eine erfindungsgemäße Darreichungsform betreffen:

| Pos. | Varianten: | A | B | C |
|---|---|---|---|---|
| | | | | |
| 1) | Wirkstoff | 150 mg | 150 mg | 150 mg |
| 2) | Siliciumdioxid, hochdispers | - | - | 10 mg |
| 3) | Mikrokristalline Cellulose | 136 mg | - | |
| 4) | Lactose | 203 mg | 336 mg | 218 mg |
| 5) | Polyvidon K 25 | 3 mg | 3 mg | 15 mg |
| 6) | Mikrokristalline Cellulose | - | - | 129 mg |
| 7) | Natriumcarboxymethylstärke | 87 mg | 90 mg | - |
| 8) | Poly(vinylpyrrolidon),querv. | - | - | 30 mg |
| 9) | Siliciumdioxid, hochdispers | 6 mg | 6 mg | 3 mg |
| 10) | Magnesiumstearat | 15 mg | 15 mg | 15 mg |
| | Endgewicht Kern | 600 mg | 600 mg | 570 mg |
| | Härte | 33 N | 15 N | 160 N |
| | Abrieb | deckeln | deckeln | < 0,5 % |
| Wrkstoff = Na-Salz des 3'-Fluor-3'-desoxy-5'-thymidylsäure-mono[3-(dodecylthio)-2-decyloxy-propyl]-esters | | | | |

Viskosität einer 2 %-igen wässrigen Lösung des Wirkstoffes: 6 mPas*sec.
Das Beispiel zeigt, daß die erfindungsgemäße IR-Darreichungsform mit Primär- und Sekundärumhüllung des Wirkstoffs nicht deckelt und einen äußerst geringen Abrieb aufweist, darüber hinaus weist sie eine entschieden höhere Härte auf.

**Patentansprüche**

1. Feste Instant-Release-Darreichungsform umfassend therapeutische Wirkstoffe oder Wirkstoffkonzentrate in einer Umhüllung bestehend aus Hilfsstoffen, wobei die Wirkstoffe oder Wirkstoffkonzentrate in wäßrigen Medien gelbildende Eigenschaften in der Art aufweisen, daß die Wirkstoffe beim Auflösen in Wasser oder wäßrigen Systemen bei Konzentrationen von weniger als 20 % eine Viskositätserhöhung der Lösung auf einen Wert von mehr als 5 mPas*s bewirken, und die Hilfsstoffe solche sind, die die Gelbildung hemmen oder kompensieren, wobei die Instant-Release-Form einen Durchmesser von mehr als 10 µm besitzt und eine Wirkstofffreisetzung von mehr als 70 % nach einer Stunde aufweist.

2. Instant-Release-Darreichungsform nach Anspruch 1, **dadurch gekennzeichnet, daß** die Wirkstoffe oder Wirkstoffkonzentrate zusätzlich zu der quellregulierenden Primärhülle von einer weiteren Umhüllung (Sekundärhülle) aus geeigneten Hilfsstoffen umgeben sind.

3. Instant-Release-Darreichungsform nach Anspruch 1, **dadurch gekennzeichnet, daß** die Hilfsstoffe aus der Gruppe der Makromoleküle, wie Polyvinylpyrrolidone, Gelatine, Gelatinederivate, Stärken, Stärke-derivate, Cellulosen, Cellulosederivate, Macrogole, Polyvinylalkohole und Polyacrylsäuren, aus der Gruppe der Zucker, Zuckeralkohole, Glyceride, Salze von Fettsäuren, Fette, Wachse, Tenside, Silikate oder hochdisperses Siliciumdioxid sind und einzeln oder in Kombination miteinander die Umhüllung bilden.

4. Instant-Release-Darreichungsform nach Anspruch 2. **dadurch gekennzeichnet, daß** die Sekundärhülle aus einzelnen Hilfsstoffen oder Kombinationen dieser gemäß Anspruch 3 besteht.

5. Instant-Release-Darreichungsform nach einem der Ansprüche 1 bis 4, **dadurch gekennzeichnet, daß** sie zusätzlich eine äußere Phase aufweist.

6. Instant-Release-Darreichungsform nach Anspruch 5, **dadurch gekennzeichnet, daß** die äußere Phase aus pharmazeutisch gebräuchlichen Hilfsstoffen wie Füllmitteln und/oder Zerfallshilfsmitteln und/oder Fließ-, Schmier- oder Trennmitteln besteht.

7. Instant-Release-Darreichungsform nach den Ansprüchen 1 und 3, **dadurch gekennzeichnet, daß** das Verhältnis von Wirkstoff oder Wirkstoffkonzentrat zu Hilfsstoff von 1:0,01 bis 1:100 beträgt.

8. Instant-Release-Darreichungsform nach Anspruch 7, **dadurch gekennzeichnet, daß** das Verhältnis von 1:0,05 bis 1:5 beträgt.

9. Instant-Release-Darreichungsform nach den Ansprüchen 1 bis 4, **dadurch gekennzeichnet, daß** das Verhältnis von Wirkstoff oder Wirkstoffkonzentrat zu Hilfsstoff von 1:0,01 bis 1:10 für die Primärhülle beträgt, und das Verhältnis für die Sekundärhülle von 1:0,1 bis 1:100, bevorzugt 1:1 bis 1:10 beträgt.

10. Instant-Release-Darreichungsform nach einem der Ansprüche 1 bis 9, **dadurch gekennzeichnet, daß** der Wirkstoffgehalt von 0,5 - 90 %, bevorzugt 5 - 50%, beträgt.

11. Instant-Release-Darreichungsform nach einem derAnsprüche 1 bis 10, **dadurch gekennzeichnet, daß** die durchschnittliche Korngroße (d') des Wirkstoffes oder Wirkstoffkonzentrates von etwa 0,01 mm bis 3 mm beträgt.

12. Instant-Release-Darreichungsform nach einem der Ansprüche 1 bis 11, **dadurch gekennzeichnet, daß** sie Wirkstoffe oder Wirkstoffkonzentrate der allgemeinen Formel 1 enthält

$$L - B - D \qquad (I),$$

wobei D eine pharmakologisch aktive Substanz. L einen lipophilen Rest und B eine die Gruppen L und D verbindende Linkergruppe darstellt.

**13.** Instant-Release-Darreichungsformen nach Anspruch 12, wobei
D eine pharmakologisch aktive Substanz.
B eine Brücke $-O-[(PO)(OH)O]_n-$ mit n = 0, 1, 2, 3.
L einen Lipidteil der allgemeinen Formel II

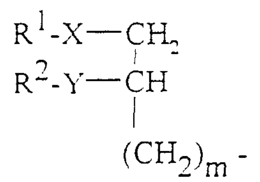

(II)

in dem

- $R^1$ eine geradkettige oder verzweigte, gesättigte oder ungesättigte Alkylkette mit 1 -30 Kohlen-stoffatomen, die gegebenenfalls ein- oder mehrfach durch $C_3$-$C_8$-Cycloalkyl- oder gegebenenfalls substituierte Phenylgruppen, Halogen, $C_1$-$C_6$-Alkoxy, $C_1$-$C_6$- Alkylmercapto-, $C_1$-$C_6$-Alkoxycarbonyl-, $C_1$-$C_6$- Alkylsulfinyl- oder $C_1$-$C_6$-Alkylsulfonylgruppen substituiert sein kann, und
- $R^2$ Wasserstoff, eine geradkettige oder verzweigte, gesättigte oder ungesättigte Alkylkette mit 1 - 20 Kohlenstoffatomen, die gegebenenfalls einoder mehrfach durch Halogen, $C_1$-$C_6$-Alkoxy-, $C_1$-$C_6$- Alkylmercapto-, $C_1$-$C_6$-Alkoxycarbonyl-, oder $C_1$-$C_6$- Alkylsulfonylgruppen substituiert sein kann,
- X einen Valenzstrich, Sauerstoff, Schwefel, Oxycarbonyl, Carbonyloxy, Carbonylamido, Amidocarbonyl, die Sulfinyl- oder die Sulfonylgruppe darstellt,
- Y einen Valenzstrich, Oxycarbonyl, Carbonyloxy, Carbonylamido, Amidocarbonyl, ein Sauerstoff- oder Schwefelatom ist, und
- m eine ganze Zahl zwischen 1 und 5 darstellt.

**14.** Instant-Release-Darreichungsform nach Anspruch 13, **dadurch gekennzeichnet, daß** sie Wirkstoffe oder Wirkstoffkonzentrate enthält. für die in Formel II

- $R^1$ eine geradkettige oder verzweigte $C_8$-$C_{15}$-Alkylkette, die durch eine $C_3$-$C_8$-Cycloalkyl- oder gegebenenfalls substituierte Phenylgruppe substituiert sein kann,
- $R^2$ eine geradkettige oder verzweigte $C_8$-$C_{15}$- Alkylkette,
- X Schwefel, Sulfinyl oder Sulfonyl und
- Y Sauerstoff

darstellen, wobei X und Y auch die aus Lecithin bekannten Carbonsäureester sein können.

**15.** Instant-Release-Darreichungsform nach Anspruch 14, **dadurch gekennzeichnet, daß**

- $R^1$ eine Nonyl-, Decyl-, Undecyl-, Dodecyl-, Tridecyl-, Tetradecyl-, Cyclohexyl-hexyl- oder Phenyl-hexylgruppe darstellt, und
- $R^2$ eine Octyl, Nonyl-, Decyl-, Undecyl-, Dodecyl-. Tridecyl- oder Tetradecylgruppe darstellt, die mit einem $C_1$-$C_6$-Alkoxy-Substituenten, wie die Methoxy-, Ethoxy-, Butoxy-, und Hexyloxygruppe oder mit einem $C_1$-$C_6$-Alkylmercaptorest wie dem Methylmercapto-, Ethylmercapto-, Butylmercapto- und Hexylmercaptorest substituiert sein kann.

**16.** Instant-Release-Darreichungsform nach Anspruch 14, **dadurch gekennzeichnet, daß** $R^1$ und $R^2$ einen Alkylrest mit 8-15 C-Atomen darstellen, X Schwefel, Y Sauerstoff und n in der Phosphatbrücke gleich 0 oder 1 ist.

**17.** Instant-Release-Darreichungsform nach Anspruch 14, **dadurch gekennzeichnet, daß** $R^1$ eine $C_1$-$C_{30}$-Alkylkette, die durch eine $C_1$-$C_6$- Alkoxy- oder eine $C_1$-$C_6$-Alkylmercaptogruppe substituiert sein kann, $R^2$ Wasserstoff, X und Y einen Valenzstrich darstellen und m gleich 1 oder 2, vorzugsweise 1, ist.

**18.** Instant-Release-Darreichungsform nach einem der Ansprüche 1 bis 17, **dadurch gekennzeichnet, daß** sie Wirkstoffe oder Wirkstoffkonzentrate aus der Gruppe der Lipidkonjugate von Nukleosiden enthält.

**19.** Instant-Release-Darreichungsform nach Anspruch 18, **dadurch gekennzeichnet, daß** sie Azidothymidin-Konjugate, Fluorthymidin-Konjugate und 5-Fluoruridin-Konjugate enthält.

**20.** Instant-Release-Darreichungsform nach einem der Ansprüche 12 - 17, **dadurch gekennzeichnet, daß** n die Zahl 0 bedeutet und D die Gruppe -P(O)(OH)-COOH bedeutet, sowie deren Ester und physiologisch verträgliche Salze.

**21.** Verfahren zur Herstellung einer Instant-Release-Darreichungsform durch Abmischen, Granulation, Sprühtrocknung, Sprüherstarrung oder Preßgranulierung von Wirkstoffen oder Wirkstoffkonzentraten, die in wäßrigen Medien gelbildende Eigenschaften haben, mit Hilfsstoffen gemäß Anspruch 3 einzeln oder in Kombination.

**22.** Verfahren nach Anspruch 21, **dadurch gekennzeichnet, daß** man in einem ersten Verfahrensschritt die Wirkstoffpartikel mit einer die Gelbildung hemmenden ersten Primärhülle von Hilfsstoffen umhüllt, in einem zweiten Verfahrensschritt die aus dem ersten Schritt resultierenden Partikel mit einer Sekundärhülle von Hilfsstoffen überzieht, und gegebenenfalls die so hergestellten Partikel zu Tabletten komprimiert, wobei die Hilfsstoffe in der Primärhülle und in der Sekundärhülle gelbildende Eigenschaften aufweisen.

**23.** Verwendung von therapeutischen Wirkstoffen oder Wirkstoffkonzentraten, die in wässrigen Medien gelbildende Eigenschaften in der Art aufweisen, daß die Wirkstoffe beim Auflösen in Wasser oder wässrigen Systemen bei Konzentrationen von weniger als 20 % eine Viskositätserhöhung der Lösung auf einen Wert von mehr als 5 mPas*s bewirken, zur Herstellung von Instant-Release-Darreichungsformen, die diese Wirkstoffe enthalten, zur Hemmung oder Kompensation der Gelbildung dieser Wirkstoffe, **dadurch gekennzeichnet, daß** man die Wirkstoffe oder Wirkstoffkonzentrate mit einer quellregulierenden Primärhülle aus Hilfsstoffen umgibt.

**24.** Verwendung nach Anspruch 23, wobei die Wirkstoffe beim Auflösen in Wasser oder wäßrigen Systemen bei Konzentrationen von weniger als 20 % eine Viskositätserhöhung der Lösung auf einen Wert von mehr als 100 mPas*s bewirken.

**25.** Verwendung nach Anspruch 23 oder 24 **dadurch gekennzeichnet, daß** man die Wirkstoffe oder Wirkstoffkonzentrate zusätzlich zu der quellregulierenden Primärhülle mit einer weiteren Umhüllung (Sekundärhülle) aus geeigneten Hilfsstoffen umgibt.

**Claims**

**1.** Solid instant-release form of administration comprising therapeutic active substances or concentrates of active substances in an envelope composed of auxiliary substances wherein the active substances or concentrates of active substances have gel-forming properties in aqueous media such that when the active substances are dissolved in water or aqueous systems at concentrations of less than 20 % they increase the viscosity of the solution to a value of more than 5 mPas*s, and the auxiliary substances are those that inhibit or compensate gel formation, wherein the instant-release form has a diameter of more than 10 µm and releases active substance by more than 70 % after one hour.

**2.** Instant-release form of administration as claimed in claim 1, wherein the active substances or concentrates of active substances are additionally surrounded by a further envelope (secondary envelope) made of suitable auxiliary substances in addition to the swell-regulating primary envelope.

**3.** Instant-release form of administration as claimed in claim 1, wherein the auxiliary substances are from the group of macromolecules such as polyvinylpyrrolidones, gelatin, gelatin derivatives, starches, starch derivatives, celluloses, cellulose derivatives, macrogols, polyvinyl alcohols and polyacrylic acids, from the group of sugars, sugar alcohols, glycerides, salts of fatty acids, fats, waxes, surfactants, silicates or highly dispersed silicon dioxide and form the envelope either individually or in combination with one another.

**4.** Instant-release form of administration as claimed in claim 2, wherein the secondary envelope is composed of individual auxiliary substances or combinations of these as claimed in claim 3.

**5.** instant-release form of administration as claimed in one of the claims 1 to 4, wherein it additionally has an outer phase.

**6.** Instant-release form of administration as claimed in claim 5, wherein the outer phase is composed of common pharmaceutical auxiliary substances such as fillers and/or agents aiding disintegration and/or flow agents, lubricants or separating agents.

**7.** Instant-release form of administration as claimed in claims 1 and 3, wherein the ratio of active substance or active substance concentrate to auxiliary substance is 1:0.01 to 1:100.

**8.** Instant-release form of administration as claimed in claim 7, wherein the ratio is 1:0.05 to 1:5.

**9.** Instant-release form of administration as claimed in claims 1 to 4, wherein the ratio of active substance or active substance concentrate to auxiliary substance is 1:0.01 to 1:10 for the primary envelope and the ratio for the secondary envelope is 1:0.1 to 1:100 preferably 1:1 to 1:10.

**10.** Instant-release form of administration as claimed in one of the claims 1 to 9, wherein the content of active substance is 0.5 - 90 %, preferably 5 - 50 %.

**11.** Instant-release form of administration as claimed in one of the claims 1 to 10, wherein the average particle size (d') of the active substance or active substance concentrate is about 0.01 mm to 3 mm.

**12.** Instant-release form of administration as claimed in one of the claims 1 to 11, wherein it contains active substances or active substance concentrates of the general formula I

$$L - B - D \qquad (I)$$

in which D represents a pharmacologically active substance, L represents a lipophilic residue and B represents a linker group linking the groups L and D.

**13.** Instant-release forms of administration as claimed in claim 12, wherein
D is a pharmacologically active substance
B is a bridge $-O-[(PO)(OH)O]_n-$ in which n = 0,1,2,3
L is a lipid moiety of the general formula II

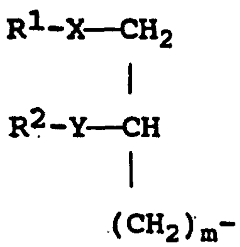

**(II)**

in which

$R^1$ is a straight-chain or branched, saturated or unsaturated alkyl chain with 1 - 30 carbon atoms which can be optionally substituted once or several times by $C_3$-$C_8$ cycloalkyl or optionally substituted phenyl groups, halogen, $C_1$-$C_6$ alkoxy, $C_1$-$C_6$ alkylmercapto, $C_1$-$C_6$ alkoxycarbonyl, $C_1$-$C_6$ alkylsulfinyl or $C_1$-$C_6$ alkylsulfonyl groups and

$R^2$ is hydrogen, a straight-chain or branched, saturated or unsaturated alkyl chain with 1 - 20 carbon atoms which can be optionally substituted once or several times by halogen, $C_1$-$C_6$ alkoxy, $C_1$-$C_6$ alkylmercapto, $C_1$-$C_6$ alkoxycarbonyl or $C_1$-$C_6$ alkylsulfonyl groups,

X represents a valency dash, oxygen, sulphur, oxycarbonyl, carbonyloxy, carbonylamido, amidocarbonyl, a sulfinyl or sulfonyl group,

Y is a valency dash, oxycarbonyl, carbonyloxy, carbonylamido, amidocarbonyl, an oxygen or sulphur atom and

m represents an integer between 1 and 5.

**14.** Instant-release form of administration as claimed in claim 13, wherein it contains active substances or active substance concentrates in which in formula II

$R^1$ represents a straight-chain or branched $C_8$-$C_{15}$ alkyl chain which can be substituted by a $C_3$-$C_8$ cycloalkyl or optionally substituted phenyl group,
$R^2$ represents a straight-chain or branched $C_8$-$C_{15}$ alkyl chain,
X represents sulphur, sulfinyl or sulfonyl and
Y represents oxygen

in which X and Y can also be the carboxylic acid esters known from lecithin.

**15.** Instant-release form of administration as claimed in claim 14, wherein

$R^1$ represents a nonyl, decyl, undecyl, dodecyl, tridecyl, tetradecyl, cyclohexyl-hexyl or phenyl-hexyl group and
$R^2$ represents an octyl, nonyl, decyl, undecyl, dodecyl, tridecyl or tetradecyl group which can be substituted by a $C_1$-$C_6$ alkoxy substituent such as a methoxy, ethoxy, butoxy or hexyloxy group or by a $C_1$-$C_6$ alkylmercapto residue such as a methylmercapto, ethylmercapto, butylmercapto or hexylmercapto residue.

**16.** Instant-release form of administration as claimed in claim 14, wherein $R^1$ and $R^2$ represent an alkyl residue with 8-15 C atoms, X denotes sulphur, Y denotes oxygen and n in the phosphate bridge equals 0 or 1.

**17.** Instant-release form of administration as claimed in claim 14, wherein $R^1$ is a $C_1$-$C_{30}$ alkyl chain which can be substituted by a $C_1$-$C_6$ alkoxy or a $C_1$-$C_6$ alkylmercapto group, $R^2$ is hydrogen, X and Y represent a valency dash and m is 1 or 2 and preferably 1.

**18.** Instant-release form of administration as claimed in one of the claims 1 to 17, wherein it contains active substances or active substance concentrates from the group of lipid conjugates of nucleosides.

**19.** Instant-release form of administration as claimed in claim 18, wherein it contains azidothymidine conjugates, fluorothymidine conjugates and 5-fluorouridine conjugates.

**20.** Instant-release form of administration as claimed in one of the claims 12 - 17, wherein n denotes the number O and D represents the group -P(O)(OH)-COOH as well as esters and physiologically tolerated salts thereof.

**21.** Process for the production of an instant-release form of administration by mixing, granulation, spray drying, spray solidification or press granulation of active substances or concentrates of active substances which in aqueous media have gel-forming properties, with auxiliary substances as claimed in claim 3 either individually or in combination.

**22.** Process as claimed in claim 21, wherein in a first process step the active substance particle is coated with a first primary envelope of auxiliary substances that inhibit gel-formation, in a second process step the particles resulting from the first step are coated with a secondary envelope of auxiliary substances and optionally the particles prepared in this way are compressed to tablets wherein the auxiliary substances in the primary envelope and in the secondary envelope have gel-forming properties.

**23.** Use of therapeutic active substances or active substance concentrates which have gel-forming properties in aqueous media such that the active substances when dissolved in water or aqueous systems at concentrations of less than 20 % increase viscosity of the solution to a value of more than 5 mPas*s, for the production of instant-release forms of administration that contain these active substances for the inhibition or compensation of the gel formation of these active substances, wherein the active substances or active substance concentrates are surrounded by a swell-regulating primary envelope of auxiliary substances.

**24.** Use as claimed in claim 23, wherein the active substances result in an increase of the viscosity of the solution to a value of more than 100 mPas*s when they are dissolved in water or aqueous systems at concentrations of less than 20 %.

**25.** Use as claimed in claim 23 or 24, wherein the active substances or active substance concentrates are additionally surrounded by a further envelope (secondary envelope) of suitable auxiliary substances in addition to the swell-regulating primary envelope.

## Revendications

**1.** Forme solide d'administration à libération instantanée comprenant des substances actives thérapeutiques ou des concentrés de substances actives dans un enrobage constitué d'adjuvants, dans laquelle les substances actives ou les concentrés de substances actives présentent des propriétés gélifiantes dans les milieux aqueux de telle sorte que les substances actives lorsqu'on les dissout dans de l'eau ou des systèmes aqueux à des concentrations inférieures à 20% provoquent une augmentation de la viscosité de la solution jusqu'à une valeur dépassant 5 mPa.s, et les adjuvants sont ceux qui inhibent ou compensent la gélification, dans laquelle la forme à libération instantanée possède un diamètre supérieur à 10 µm et présente une libération de substances actives supérieure à 70% après une heure.

**2.** Forme d'administration à libération instantanée selon la revendication 1, **caractérisée en ce que** les substances actives ou les concentrés de substances actives en plus de l'enveloppe primaire régulant le gonflement, sont entourées par un enrobage supplémentaire (enveloppe secondaire) constitué d'adjuvants appropriés.

**3.** Forme d'administration à libération instantanée selon la revendication 1, **caractérisée en ce que** les adjuvants sont choisis dans le groupe des macromolécules, comme la polyvinylpyrrolidone, la gélatine, les dérivés de gélatine, les amidons, les dérivés d'amidon, les celluloses, les dérivés de cellulose, les macrogels, les poly(alcool vinylique)s et les poly(acide acrylique)s, dans le groupe des sucres, les alcools de sucre, les glycérides, les sels d'acides gras, les graisses, les cires, les tensioactifs, les silicates ou le dioxyde de silicium à forte dispersion et forment individuellement ou associés les uns aux autres, l'enrobage.

**4.** Forme d'administration à libération instantanée selon la revendication 2, **caractérisée en ce que** l'enveloppe secondaire est constituée d'adjuvants individuels ou d'associations de ces derniers selon la revendication 3.

**5.** Forme d'administration à libération instantanée selon l'une quelconque des revendications 1 à 4, **caractérisée en ce qu'**elle présente en outre une phase externe.

**6.** Forme d'administration à libération instantanée selon la revendication 5, **caractérisée en ce que** la phase externe est constituée d'adjuvants à usage pharmaceutique tels que les charges et/ou les adjuvants de délitement et/ou les agents rhéologiques, les lubrifiants ou les agents séparateurs.

**7.** Forme d'administration à libération instantanée selon les revendications 1 et 3, **caractérisée en ce que** le rapport de la substance active ou du concentré de substance active à l'adjuvant est de 1:0,01 à 1:100.

**8.** Forme d'administration à libération instantanée selon la revendication 7, **caractérisée en ce que** le rapport est de 1:0,05 à 1:5.

**9.** Forme d'administration à libération instantanée selon les revendications 1 à 4, **caractérisée en ce que** le rapport de la substance active ou du concentré de substance active à l'adjuvant, est de 1:0,01 à 1:10 pour l'enveloppe primaire, et le rapport pour l'enveloppe secondaire est de 1:0,1 à 1:100, de préférence de 1:1 à 1:10.

**10.** Forme d'administration à libération instantanée selon l'une des revendications 1 à 9, **caractérisée en ce que** la teneur en substance active est de 0,5 à 90%, de préférence de 5 à 50%.

**11.** Forme d'administration à libération instantanée selon l'une quelconque des revendications 1 à 10, **caractérisée en ce que** la granulométrie moyenne (d') de la substance active ou du concentré de substance active est d'environ 0,1 mm à 3 mm.

**12.** Forme d'administration à libération instantanée selon l'une quelconque des revendications 1 à 11, **caractérisée en ce qu'**elle contient des substances actives ou des concentrés de substances actives de formule générale I

$$L - B - D \qquad (I)$$

dans laquelle D représente une substance active sur le plan pharmacologique, L, un résidu lipophile et B, un groupe lieur liant les groupes L et D.

**13.** Forme d'administration à libération instantanée selon la revendication 12, dans laquelle
D représente une substance active sur le plan pharmacologique,
B représente un pont $-O-[(PO)(OH)O]_n-$, avec n = 0, 1, 2, 3,
L représente une portion lipidique de formule générale II

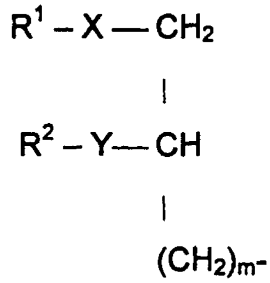

(II)

dans laquelle

- $R^1$ représente une chaîne alkyle insaturée ou saturée, à chaîne droite ou ramifiée, ayant 1 à 30 atomes de carbone, qui peut être éventuellement substituée une ou plusieurs fois par des groupes cycloalkyle en $C_3$ à $C_8$ ou groupes phényle éventuellement substitués, halogéno, alcoxy en $C_1$ à $C_6$, alkylmercapto en $C_1$ à $C_6$, (alcoxy en $C_1$ à $C_6$)-carbonyle, alkylsulfinyle en $C_1$ à $C_6$, ou alkylsulfonyle en $C_1$ à $C_6$, et
- $R^2$ représente un atome d'hydrogène, une chaîne alkyle saturée ou insaturée, à chaîne droite ou ramifiée, ayant 1 à 20 atomes de carbone, qui peut être éventuellement substituée une ou plusieurs fois par des groupes halogéno, alcoxy en $C_1$ à $C_6$, alkylmercapto en $C_1$ à $C_6$, (alcoxy en $C_1$ à $C_6$)-carbonyle ou alkylsulfonyle en $C_1$ à $C_6$,
- X représente une liaison de valence, un atome d'oxygène, de soufre, un groupe oxycarbonyle, carbonyloxy, carbonylamido, amidocarbonyle, le groupe sulfinyle ou le groupe sulfonyle,
- Y représente une liaison de valence, un groupe oxycarbonyle, carbonyloxy, carbonylamido, amidocarbonyle, un atome d'oxygène ou de soufre, et
- m représente un nombre entier compris entre 1 et 5.

**14.** Forme d'administration à libération instantanée selon la revendication 13, **caractérisée en ce qu'**elle contient des substances actives ou des concentrés de substances actives, pour lesquels dans la formule II

- $R^1$ représente une chaîne alkyle en $C_8$ à $C_{15}$ chaîne droite ou ramifiée, qui peut être substituée par un groupe cycloalcyle en $C_3$ à $C_8$ ou éventuellement par un groupe phényle substitué,
- $R^2$ représente une chaîne alkyle en $C_8$ à $C_{15}$;
- X représente un atome de soufre, un groupe sulfinyle ou sulfonyle et
- Y représente un atome d'oxygène
- X et Y pouvant également être les esters d'acide carboxylique connus issus de la lécithine.

**15.** Forme d'administration à libération instantanée selon la revendication 14, **caractérisée en ce que**

- $R^1$ représente un groupe nonyle, décyle, undécyle, dodécyle, tridécyle, tétradécyle, cyclohexyl-hexyle ou phényl-hexyle et
- $R^2$ représente un groupe octyle, nonyle, décyle, undécyle, dodécyle, tridécyle, ou tétradécyle, qui peut être substitué par un substituant alcoxy en $C_1$ à $C_6$, tel que le groupe méthoxy, éthoxy, butoxy et hexyloxy ou

par un résidu alkylmercapto en $C_1$ à $C_6$, tel que le résidu méthylmercapto, éthylmercapto, butylmercapto et hexylmercapto.

**16.** Forme d'administration à libération instantanée selon la revendication 14, **caractérisée en ce que** $R^1$ et $R^2$ représentent un résidu alkyle ayant 8 à 15 atomes de C, X représente un atome de soufre, Y un atome d'oxygène et n dans le pont phosphate est égale à 0 ou à 1.

**17.** Forme d'administration à libération instantanée selon la revendication 14, **caractérisée en ce que** $R^1$ représente une chaîne alkyle en $C_1$ à $C_{30}$, qui peut être substituée par un groupe alcoxy en $C_1$ à $C_6$, ou un groupe alkylmercapto en $C_1$ à $C_6$, $R^2$ représente un atome d'hydrogène, X et Y représentent une liaison de valence et m est égal à 1 ou à 2, vaut de préférence 1.

**18.** Forme d'administration à libération instantanée selon l'une quelconque des revendications 1 à 17, **caractérisée en ce qu'**elle contient des substances actives ou des concentrés de substances actives choisis dans le groupe des conjugués lipidiques de nucléosides.

**19.** Forme d'administration à libération instantanée selon la revendication 18, **caractérisée en ce qu'**elle contient des conjugués d'azidothymidine, des conjugués de fluorothymidine, et des conjugués de 5-fluoro-uridine.

**20.** Forme d'administration à libération instantanée selon l'une quelconque des revendications 12 à 17, **caractérisée en ce que** n représente le nombre 0 et D désigne le groupe -P(O)(OH)-COOH, ainsi que leurs esters et sels acceptables sur le plan physiologique.

**21.** Procédé de préparation d'une forme d'administration à libération instantanée par mélange, granulation, séchage par pulvérisation, solidification par pulvérisation, ou granulation à la presse, de substances actives ou de concentrés de substances actives qui présente des propriétés gélifiantes dans des milieux aqueux, individuellement ou en association avec des adjuvants selon la revendication 3.

**22.** Procédé selon la revendication 21, **caractérisé en ce que** dans une première étape de procédé, on enrobe les particules de substances actives d'une première enveloppe primaire d'adjuvants, inhibitrice de la gélification, dans une deuxième étape de procédé, on recouvre les particules résultant de la première étape, d'une enveloppe secondaire d'adjuvants et on comprime éventuellement les particules ainsi préparées en comprimés, les adjuvants dans l'enveloppe primaire et dans l'enveloppe secondaire présentant des propriétés gélifiantes.

**23.** Utilisation de substances actives thérapeutiques ou de concentrés de substances actives qui présentent des propriétés gélifiantes dans des milieux aqueux de sorte que les substances actives lorsqu'on les dissout dans de l'eau ou des systèmes aqueux à des concentrations inférieures à 20%, provoquent une augmentation de la viscosité de la solution jusqu'à une valeur dépassant 5 mPa.s, en vue de préparer des formes d'administration à libération instantanée, qui contiennent ces substances actives, pour inhiber ou compenser la gélification de ces substances, **caractérisée en ce que** l'on enveloppe les substances actives et les concentrés de substances actives, d'une enveloppe primaire à base d'adjuvants régulant le gonflement.

**24.** Utilisation selon la revendication 23, dans laquelle les substances actives lorsqu'on les dissout dans de l'eau ou des systèmes aqueux à des concentrations inférieures à 20% provoquent une augmentation de la viscosité de la solution jusqu'à une valeur dépassant 100 mPa.s.

**25.** Utilisation selon la revendication 23 ou 24, **caractérisée en ce que** l'on enveloppe les substances actives ou les concentrés de substances actives, d'un enrobage supplémentaire (enveloppe secondaire) à base d'adjuvants appropriés, en plus de l'enveloppe primaire régulant le gonflement.

**Abb. 1**

Einbettung des Wirkstoffs in Primär- und Sekundärhülle